# EUROPÄISCHE PATENTSCHRIFT

(11) **EP 1 747 792 B1**
(45) Veröffentlichungstag und Bekanntmachung des Hinweises auf die Patenterteilung: **10.09.2008**
(21) Anmeldenummer: 06015559.5
(22) Anmeldetag: 26.07.2006
(51) Int. Cl.: A61M 16/10, A61M 16/04

(54) **Feuchte- und Wärmeaustauscher mit Sprechfunktion**
Heat and moisture exchanger with speaking function
Échangeur de chaleur et d'humidité avec fonction vocale

(30) Priorität: 26.07.2005 DE 102005034762
(43) Veröffentlichungstag der Anmeldung: 31.01.2007
(73) Patentinhaber: Andreas Fahl Medizintechnik-Vertrieb GmbH, 51149 Köln (DE)
(72) Erfinder: Fahl, Andreas, 51107 Köln (DE)
(74) Vertreter: Maxton Langmaack & Partner

(56) Entgegenhaltungen:
- EP-A2- 0 265 163
- WO-A-99/60954
- WO-A-20/05058403
- DE-A1- 3 900 183
- DE-U1-9202004 008 95
- DE-U1-6202005 002 53

## Beschreibung

Die vorliegende Erfindung betrifft einen Feuchte- und Wärmeaustauscher mit Sprechfunktion, insbesondere für eine einmalige Benutzung, mit einem Filtergehäuse und einem in diesem zumindest teilweise angeordneten Filterkörper, wobei das Filtergehäuse ein auf seiner distalen Seite angeordnetes Sprechventil aufweist, sowie dessen Verwendung.

Bei operativen Eingriffen im oberen Atemtrakt kann das Anlegen einer künstlichen Atemöffnung (Tracheostoma) in der Luftröhre notwendig sein, damit unter Umgehung von Mundraum und Kehlkopf Luft direkt in die Lunge eingeatmet werden kann. Bei Tracheotomierten werden üblicherweise Filtersysteme eingesetzt, welche entweder aus einem Pflaster mit einem eingesetzten Filter oder aus einer üblicherweise selbstklebenden Basisplatte - in aller Regel aus Kunststoff gebildet -, in welche Filter unterschiedlichster Art einsetzbar sind.

Zu den Filtersystemen, welche für laryngo-tracheale Hilfsmittel wie Trachealkanülen und Filtersysteme mit Pflaster- oder Basisplatte eingesetzt werden, zählen die so genannten Feuchte- und Wärmeaustauscher, auch künstliche Nase genannt. Diese dienen dazu, die bei Tracheotomierten, aber auch laryngektomierten Patienten fehlenden Regulationsmechanismen zur Erwärmung und Befeuchtung der Atemluft nachzubilden und ein Inkontaktbringen der Luftröhre mit trockener, kalter und nicht gefilterter Luft zu vermeiden. Denn durch die hierdurch hervorgerufene Reizung tritt eine erhöhte Schleimproduktion mit der nachfolgenden Gefahr der Verborkung auf. Durch Feuchte- und Wärmeaustauscher wird nun die eingeatmete Luft angefeuchtet, erwärmt und gleichzeitig gefiltert. Hierdurch wird die vorstehend erwähnte Borkenbildung weitgehend vermieden. Dabei hilft das regelmäßige Tragen der künstlichen Nase insbesondere bei starker Sekretabsonderung, da durch das Anfeuchten der Schleimhäute in der Luftröhre die Sekretproduktion vermindert wird.

Feuchte- und Wärmeaustauscher für Tracheotomierte als auch Laryngektomierte weisen einen Filterkörper auf ― in aller Regel aus Papier oder Schaumstoff gebildet ― durch welchen die ein- und ausgeatmete Luft geleitet wird. Beim Ausatmen hält der Filterkörper Feuchtigkeit zurück, welche dann beim Einatmen in die Luftröhre transportiert wird. Aus dem Stand der Technik bekannte künstliche Nasen, auch HME (Humid and Moisture Exchanger) genannt, gibt es in einer großen Vielzahl unterschiedliche Ausbildungen für unterschiedliche Adapter. Der Universaladapter gemäß DIN EN ISO 5356/1 weist dabei einen Durchmesser von 15 mm auf, es sind jedoch aus dem Stand der Technik auch weitere Adapter mit insbesondere größeren Durchmessern bekannt. Der Universaladapter wird insbesondere bei Trachealkanülen eingesetzt, wohingegen beispielsweise Filtersysteme, insbesondere mit einer selbstklebenden Basisplatte oder mit einem Pflaster ausgebildet, einen größeren Adapterdurchmesser aufweisen.

Feuchte- und Wärmeaustauscher können darüber hinaus noch mit einer Sprechfunktion ausgestattet sein. So offenbart beispielsweise DE 39 00 183 C2 ein Atemventil zur Anwendung als Sprechventil in Tracheotomieschläuchen, wobei dieses Atemventil zusätzlich einen Filter aufweist, welcher als Feuchte- und Wärmeaustauscher dient. Dabei ist ein gegen eine Schulter eines Ventilgehäuses anlegbarer Filterkörper hinter einer Membran herausnehmbar angeordnet, wobei der Filterkörper von einer in das Ventilgehäuse einsetzbaren schlüsselförmigen Kappe mit einem durchbrochenen Boden und einem sich zum Filterkörper hin erstreckenden mittleren Zapfen in seinem Sitz gehalten ist. Die Membran ist dabei an dem mittleren Zapfen der Kappe befestigt. Der Aufbau des in der DE 39 00 183 C2 offenbarten Atemventils ist somit ausgesprochen kompliziert, weshalb dieses in der Herstellung relativ teuer ist. Die Sprechfunktion wird dadurch ermöglicht, dass die in dem Ventilgehäuse angeordnete bewegliche Membran als Rückschlagventil funktioniert, welches beim Einatmen öffnet und beim Ausatmen oder Sprechen schließt.

Des Weiteren ist aus der DE 102 46 929 A1 eine Sprechventilanordnung bekannt, welche allerdings nicht die Funktion eines Feuchte- und Wärmeaustauschers aufweist. Dabei weist die dort offenbarte Sprechventilanordnung eine Ventilklappe und ein Ventilgehäuse auf, welches eine patientenseitige und eine patientenabgewandte Öffnung und einen dazwischenliegenden Innenraum aufweist, wobei im Innenraum ein lösbares Sprechventil angeordnet ist, das einen Ventilkörper und die mit dem Ventilkörper schwenkbar verbundene Ventilklappe umfasst, wobei die Ventilklappe an der patientenabgewandten Öffnung angeordnet ist und diese bei Expiration abdeckt. Das Sprechventil ist aus einem Gehäuse gebildet, welches wiederum im eigentlichen Ventilgehäuse aufgenommen ist. Dieses Gehäuse liegt auf einer im eigentlichen Ventilgehäuse gebildeten Schulter auf, und weist für sich einen Innenraum auf, welcher eine Bewegung der Ventilklappe bei Einatmen ermöglicht. Somit ist die in der DE 102 46 929 A1 offenbarte Sprechventilanordnung wiederum relativ kompliziert ausgebildet, andererseits weist diese nicht die Funktion eines Feuchte- und Wärmeaustauschers auf.

DE 20 2005 002 536 U1 offenbart einen Feuchte- und Wärmeaustauscher ohne Sprechfunktion, mit einem Sicherheitsventil, insbesondere für Laryngektomierte oder Tracheotomierte, mit einem Filterteil, umfassend einen Filterkörper und ein Filtergehäuse, und einem rohrförmig ausgebildeten Ansatzteil, wobei der Filterkörper zumindest teilweise durch eine Teilfläche der distalen Stirnseite des rohrförmigen Ansatzteils gehalten ist.

WO 2005/058403 A1 offenbart einen alternativen, komplex ausgebildeten Feuchte- und Wärmeaustauscher ohne Sprechfunktion, mit einem Sicherheitsventil, bei welchem zwei Filterkörper in etwa senkrecht zu einer Trachealkanüle angeordnet sind.

WO 99/60954 offenbart einen Feuchte- und Wärmeaustauscher mit Sprechfunktion und einem ringförmig ausgebildeten Filterkörper, welcher durch die Gehäusewandungen des Austauschers selbst gehalten sind.

EP 0 265 163 A2 offenbart einen kompliziert ausgebildeten Feuchte- und Wärmeaustauscher, welcher keine Sprechfunktion aufweist, bei welchem durch eine Quetschung zweier Gehäuseteile ein Filterkörper gehalten ist.

DE 20 2004 008 959 U1 offenbart einen Feuchte- und Wärmeaustauscher, wobei in einem als Kassette ausgebildeten Gittergehäuse mit Deckel ein profilierter Schaumstoffzuschnitt für die Kombination mit einem Sprechventil deponiert ist und der Deckel mit Hilfe einer Klammer am Sprechventil montiert oder demontiert wird.

Aufgabe der vorliegenden Erfindung ist es daher, einen Feuchte- und Wärmeaustauscher mit Sprechfunktion zur Verfügung zu stellen, welcher einfachst aufgebaut ist.

Diese Aufgabe wird gemäß der vorlegenden Erfindung gelöst durch einen Feuchte- und Wärmeaustauscher mit Sprechfunktion, wie bean Ansprucht Durch die Anordnung eines relativ einfach auszubildenden Haltemittels im Inneren des Filtergehäuses wird der Filterkörper sicher gehalten, so dass dieser sich weder beim Ein- noch beim Ausatmen bewegen kann. Hierdurch ist eine ausreichende Bewegungsfreiheit für das Sprechventil beim Ein- und Ausatmen sichergestellt. Der Filterkörper ist dabei vorzugsweise aus einem geeigneten porigen Schaumstoff gebildet, welcher weiter bevorzugt elastische Eigenschaften aufweist.

Vorzugsweise ist das Haltemittel auf der Innenumfangsfläche und dem Boden des Filtergehäuses angeordnet. Der Boden muss selbstverständlich luftdurchlässig sein, um ein Ein- oder Ausatmen mittels des erfindungsgemäßen Feuchte- und Wärmeaustauschers zu ermöglichen. Dies kann beispielsweise dadurch erreicht werden, dass der Boden eine Vielzahl beispielsweise kreisrund ausgebildeter Öffnungen, somit eine perforierte Bodenplatte, aufweist, es kann aber beispielsweise auch alternativ vorgesehen sein, hier als proximales Haltemittel für den Filterkörper den Boden als Kreuz, gebildet aus zwei Streben, auszubilden. Die Haltemittel sind dann auf der dem Filterkörper zugewandten Seite der Streben und/oder zusätzlich zu einem ringförmig ausgebildeten Boden auf dessen dem Filterkörper zugewandten Seite angeordnet, wobei der Bodenring in seiner Mitte das Filterkreuz tragen kann. Der Bodenring kann jedoch auch derart ausgebildet sein, dass dieser ausgesprochen schmal ausgebildet ünmittelbar anschließend an das Filtergehäuse angeordnet ist, so dass die Streben des Kreuzes relativ lang ausgebildet sind, und im Wesentlichen allein den Filterkörper tragen.

In einer bevorzugten Ausführungsform ist das Haltemittel als auf der Innenumfangsfläche des Filtergehäuses angeordneter umlaufender Ringwulst ausgebildet. Der Ringwulst kann dabei auch mehrfach unterbrochen sein. Weiter bevorzugt ist das Haltemittel zapfenförmig ausgebildet. Dabei können an der Innenumfangsfläche oder auf dem Boden des Filtergehäuses ein, bevorzugt mindestens zwei zapfenförmige Haltemittel angeordnet sein.

Um eine Bewegung des Filterkörpers im Filtergehäuse in Längsrichtung auf das Sprechventil zu sicher zu vermeiden, ist es dabei vorteilhaft, wenn mindestens ein Haltemittel auf der Innenumfangsfläche des Filtergehäuses angeordnet ist.

Weiter bevorzugt ist das Haltemittel sich auf den Filterkörper hin verjüngend ausgebildet, bevorzugt spitz zulaufend. Dabei kann das Haltemittel auch derart ausgebildet werden, dass dieses hakenförmig ausgebildet ist. In diesem Fall kann eine Verschiebung des Filterkörpers im Filtergehäuse auf das Sprechventil hin sicher auch durch am Boden des Filtergehäuses angeordnete hakenförmig ausgebildete Haltemittel vermieden werden. Besonders bevorzugt ist es, dass das Haltemittel, gleich ob auf dem Boden bzw. an der Innenumfangsfläche des Filtergehäuses angeordnet, in den Filterkörper selbst eingreift. Unter Eingreifen im Sinne der vorliegenden Erfindung wird dabei insbesondere verstanden, dass aufgrund der bevorzugt elastischen, auf jeden Fall kompressiblen Ausbildung des Filterkörpers, der in jedem Falle stets aus einem weicheren Material besteht als das Haltemittel, dieses Haltemittel zumindest teilweise in den Filterkörper selbst eindringen bzw. durch eine zumindest partielle Zusammenpressung desselben diesen halten und so einen sicheren Halt desselben im Filtergehäuse vermitteln kann. Eine Verschiebung des Filterkörpers entlang der Längsachse als auch der Querachse des Filtergehäuses ist dadurch sicher vermieden. Dabei liegen bevorzugt sämtliche Haltemittel innerhalb des von dem Filterkörper abgedeckten Bereiches. Hierdurch kann eine sehr einfache Ausbildung des erfindungsgemäßen Feuchte- und Wärmeaustauschers erzielt werden, da insbesondere die Vorsehung einer Schulter bzw. Vor- und Rücksprüngen an der Innenseite des rohrförmig ausgebildeten Filtergehäuses entfällt.

In einer weiter bevorzugten Ausführungsform des erfindungsgemäßen Feuchte- und Wärmeaustauschers weist das Sprechventil eine in einem Deckelteil angeordnete Membran auf. Die Membran kann dabei insbesondere aus Silikon oder hierzu ähnlichem Materialien hergestellt sein. Der Deckelteil weist vorzugsweise eine Öffnung auf, welche vollständig durch die Membran überdeckt wird. Die Überdeckung durch die Membran ist dabei bezogen auf den Zustand eines nicht in Benutzung befindlichen Feuchte- und Wärmeaustauschers. Die Membran überdeckt dabei die Öffnung um mehr als etwa 5 %, weiter bevorzugt um mehr als etwa 10 %, noch weiter bevorzugt um mehr als etwa 15 %, bezogen auf den Innendurchmesser der Öffnung im Deckelteil. Der Deckelteil ist vorzugsweise lösbar mit dem Filtergehäuse verbunden, wobei die Verbindung formschüssig, insbesondere als Druckverbindung ausgeführt ist. Das Deckelteil weist dabei eine geringe Bauhöhe auf, so dass jedoch gleichwohl zwischen der Innenseite des Deckelteiles bzw. der Membran und der dem Deckelteil zugewandten Seite des Filterkörpers ein Freiraum gebildet ist.

Die Membran ist auf der Innenseite des Deckelteiles an mindestens einem Befestigungspunkt, bevorzugt mindestens zwei Befestigungspunkten gehalten. Dabei sind dann beide Befestigungspunkte benachbart zueinander angeordnet, so dass beim Einatmen mittels eines sich in Benutzung befindlichen Feuchte- und Wärmeaustauschers die Membran sich in Richtung auf den gebildeten Freiraum hin bewegen und hierdurch Luft einlassen kann. Dien Befestigungspunkte sind dabei bevorzugt als Klebeverbindung ausgeführt, es können jedoch auch jegliche andere Arten einer Befestigung der Membran an der Deckelinnenseite, aber alternativ auch am Innenrand des Deckelteiles vorgesehen werden, einschließlich mechanischer Verbindungsarten und eine Verbindung durch Verschweißung.

Vorzugsweise ist die Membran mit einer unebenen Oberfläche versehen, wobei die unebene Oberfläche mindestens auf einer Seite der Membran, bevorzugt auf beiden Seiten der Membran ausgebildet ist. Dabei ist bevorzugt eine gleichmäßigere Riffelung über die gesamte Membranoberfläche vorgesehen, wobei die Unebenheit zum Beispiel in Form einer linearen oder gekreuzten Riffelung ausgeführt sein kann. Hierdurch wird insbesondere sichergestellt, dass die Membran mit Ihrer der distalen Seite des Feuchte- und Wärmeaustauschers zugewandten Seite nicht auf der Innenseite des Deckelteiles des Sprechventils zu fest anliegt, was insbesondere auch bei größerer Außenfeuchtigkeit oder aber bei Abgabe von Feuchtigkeit aus dem Feuchte- und Wärmeaustauschers der Fall sein kann.

In einer bevorzugten Ausführungsform des erfindungsgemäßen Feuchte- und Wärmeaustauschers ist über dem im Filtergehäuse angeordneten Filterkörper ein Freiraum gebildet, der der Membran eine auf den Fitterkörper zugerichtete Bewegung beim-Einatmen ermöglicht. Dieser Freiraum erstreckt sich einerseits innerhalb des Filtergehäuses, andererseits jedoch innerhalb des Gehäuses des Deckelteiles, welches in aller Regel jedoch nur eine geringe Bauhöhe aufweist. Der Freiraum ist dabei derart ausgestaltet, dass eine Ablenkung der Membran, ausgehend von einer durch die Deckelteilinnenseite definierten Ebene, von mindestens 15%, weiter bevorzugt mindestens 20 %, noch weiter bevorzugt mindestens 30% ermöglicht ist.

Diese und weitere Vorteile werden anhand der nachfolgenden Figuren näher erläutert. Es zeigen:
- Fig. 1:: Eine Seitenansicht eines erfindungsgemäßen Feuchte- und Wärmeaustauschers;
- Fig. 2:: eine Draufsicht auf den Kopfbereich des erfindungsgemäßen Feuchte- und Wärmeaustauschers gemäß Fig. 1;
- Fig. 3:: eine perspektivische Unteransicht von schräg unten des erfindungsgemäßen Feuchte- und Wärmeaustauschers gemäß Fig. 1; sowie
- Fig. 4:: eine perspektivische Ansicht von schräg oben des erfindungsgemäßen Feuchte- und Wärmeaustauschers gemäß Fig. 1.

Zunächst sei bemerkt, dass die in den Figuren gezeigten Merkmale nicht auf die einzelnen Ausgestaltungen beschränkt sind. Vielmehr sind die jeweils in der Beschreibung einschließlich der Figurenbeschreibung und Zeichnungen angegebenen Merkmale zur Weiterbildung miteinander kombinierbar. Im Übrigen werden gleich Bezugszeichen für gleiche Merkmale verwendet.

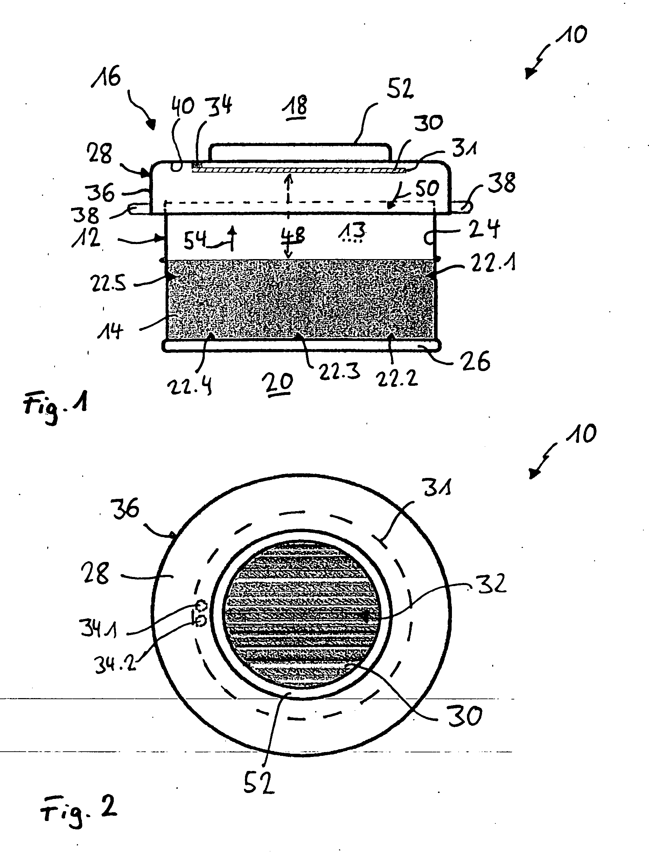

Fig. 1 zeigt nun einen erfindungsgemäßen Feuchte- und Wärmeaustauscher 10 mit einem Filtergehäuse 12 und einem in diesem aufgenommen Filterkörper 14. Des Weiteren weist der Feuchte- und Wärmeaustauscher 10 ein Sprechventil 16 auf, gebildet aus einem Dekkelteil 28 und einem auf der Innenseite 40 des Deckelteiles 28 mittels eines Befestigungspunktes 34, ausgeführt als Klebeverbindung, angeordneten Membran 30 mit einem Membranrand 31.

Das Sprechventil 16 ist auf der distalen Seite 18 des Feuchte- und Wärmeaustauscher 10 angeordnet, wohingegen der Filterkörper 14 im Filtergehäuse 12 auf der proximalen Seite 20 des Feuchte- und Wärmeaustauschers 10 angeordnet ist.

Der Deckelteil 28 des Sprechventils 16 weist einen Rand 36 und einen an diesem umlaufend ausgebildeten Ansatz 38 auf. Dieser Ansatz 38 ist bei den Fig. 2 bis 4 der Einfachheit halber nicht gezeigt. Der Ansatz 38 dient dabei insbesondere der leichteren Lösbarkeit des Sprechventils 16 von dem Filtergehäuse 12. Der Deckelteil 28 weist des Weiteren auf seiner distalen Seite 18 einen Ansatz 52 auf, in welchem eine in der Fig. 2 gezeigte Öffnung 32, welche kreisförmig ausgebildet ist, eingelassen ist.

Auf einer Innenumfangsfläche 24 des Filtergehäuses 12 angeordnet sind zwei Haltemittel 22.1 und 22.5, welche spitz zulaufend in den Filterkörper 14 hineinragen. Diese sind dabei zapfenförmig ausgebildet. Des Weiteren sind auf dem Boden 26 des Filtergehäuses 12 weitere Haltemittel 22.2, 22.3 und 22.4 ausgebildet, welche ebenfalls spitz zulaufend in den Filterkörper 14 hineinragen. Der Filterkörper 14 ist dabei aus einem elastischen Schaumstoffmaterial mit feinen Poren ausgebildet, welches geeignet ist, Atemluft zu filtrieren und anzufeuchten. Durch die auf dem Boden 26 angeordneten Haltemittel 22.2, 22.3 und 22.4 sowie die auf der Innenumfangsfläche 24 des Filtergehäuses 12 angeordneten Haltemittel 22.1 und 22.5 ist insbesondere eine Bewegung des Filterkörpers 14 insbesondere in Richtung eines Pfeils 54 auf das Sprechventil 16 hin sicher vermieden. Der Filterkörper 14 füllt das Filtergehäuse 12 nicht zur Gänze aus, sondern nur zu etwa 60 bis 70 %. Hierdurch ist ein Freiraum 48 gebildet, in welchen bei Einatmung durch den Feuchte- und Wärmeaustauscher 10 sich die Membran 30 in Richtung eines Pfeils 50 hinein erstreckt. Der Freiraum 48 wird ergänzt durch einen weiteren freien Raum, welcher durch die Bauhöhe des Deckelteiles 28 des Sprechventils 16 definiert wird. Das Sprechventil 16 könnte jedoch auch extrem flach ausgebildet werden, so dass hier kein zusätzlicher freier Raum für eine ausreichende Bewegung der Membran 30 zur Verfügung steht. In diesem Fall würde der gesamte für die Bewegung der Membran 30 benötigte Freiraum durch den Freiraum 48 zur Verfügung gestellt werden.

Fig. 2 zeigt nun in einer Draufsicht den Feuchte- und Wärmeaustauscher 10 gemäß Fig. 1. Deutlich ist zu erkennen, dass die Membran 30 die Öffnung 32 vollständig abdeckt. Dabei ist der Durchmesser der Membran 30 größer als der Durchmesser der Öffnung, aber auch größer als der Außendurchmesser des Ansatzes 52 gewählt. Gemäß Fig. 2 liegt der äußere Membranrand 30 im Bereich zwischen dem Außenrand des Ansatzes 52 und dem Rand 36 des Deckelteils 28. Wie Fig. 2 zu entnehmen ist, ist die Membran 30 über zwei Befestigungspunkte 34.1 und 34.2, welche als Klebeverbindungen ausgeführt sind, auf der Deckelinnenseite 40 des Deckelteils 28 des Sprechventils 16 gehalten. Dabei sind die beiden Befestigungspunkte 34.1 und 34.2 unmittelbar benachbart angeordnet, so dass eine Öffnung der Membran 30 bei Einatmen durch Klappen in Richtung des Pfeils 50 gemäß Fig. 1 ermöglicht ist.

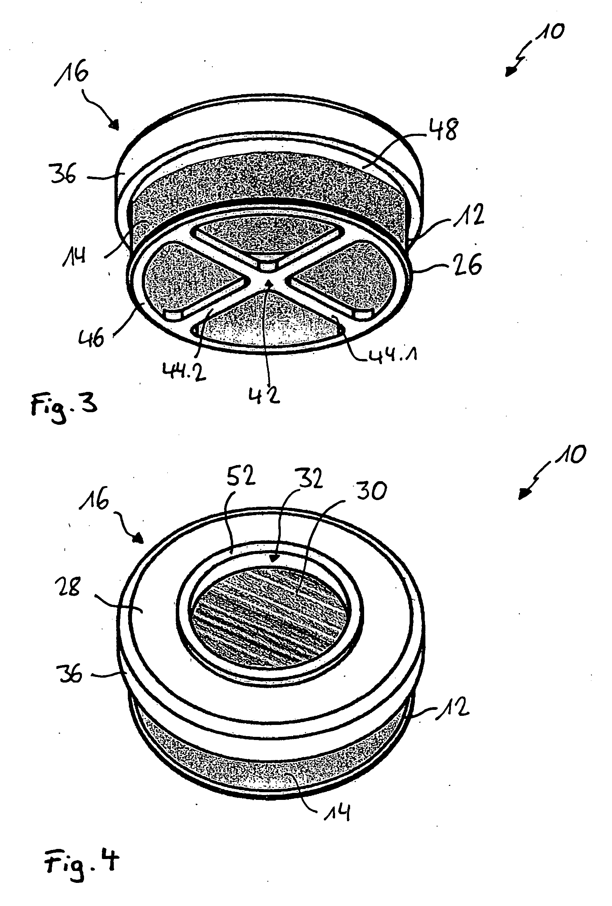

Fig. 3 zeigt nun in einer perspektivischen Ansicht von schräg unten den Feuchte- und Wärmeaustauscher 10. Dabei ist besonders gut zu erkennen, dass der Boden 26 hier nicht als durchgehende Bodenplatte ausgebildet ist, sondern vielmehr einen Bodenring 46 umfasst, welcher unmittelbar der rohrförmigen Wand des Filtergehäuses 12 angeschlossen ist. Dabei ist dieser Bodenring 46 relativ schmal ausgeführt, und weist in seiner Mitte ein proximales Haltemittel für den Filterkörper 14 auf, welches kreuzförmig mit zwei Streben 44.1 und 44.2 ausgebildet ist. Auf der dem Filterkörper 14 zugewandten Seite weisen die Streben 44.1 und 44.2 die Haltemittel 22.2, 22.3 und 22.4 gemäß Fig. 1 auf. Alternativ wäre beispielsweise möglich, den Boden 26 als perforierte Lochbodenplatte auszubilden. Der Boden 26 ist Teil des Filtergehäuses 12.

Fig. 4 schließlich zeigt in einer perspektivischen Ansicht von schräg oben den Feuchte- und Wärmeaustauscher 10, wobei hier gut die Öffnung 32 zu erkennen ist, welche innerhalb des Ansatzes 52 gebildet ist. Diese Öffnung 32 wird vollständig von der Membran 30 überdeckt.

Durch die vorliegende Erfindung wird somit vorteilhafterweise ein Feuchte- und Wärmeaustauscher zur Verfügung gestellt, welcher gleichzeitig eine Sprechfunktion aufweist, und ausgesprochen einfach und kostengünstig hergestellt werden kann. Er kann insbesondere auch als Wegwerfprodukt ausgebildet sein, und dabei einen erheblichen praktischen Nutzen für Tracheotomierte aufweisen.

## Patentansprüche

1. Feuchte- und Wärmeaustauscher (10) mit Sprechfunktion, insbesondere für eine einmalige Benutzung, mit einem Filtergehäuse (12) mit einem Boden (26) und einem in diesem zumindest teilweise angeordneten Filterkörper (14), wobei das Filtergehäuse ein auf seiner distalen Seite (18) angeordnetes Sprechventil (16) aufweist, **dadurch gekennzeichnet, dass** im Inneren (13) des Filtergehäuses (12) mindestens ein Haltemittel (22) zur Vermittlung eines Haltes für den Filterkörper (14) angeordnet ist, wobei das Haltemittel (22) auf einer Innenumfangsfläche (24) des Filtergehäuses (12) angeordnet ist und in den Filterkörper (14) eingreift, wobei das Sprechventil (16) eine in einem Deckelteil (28) angeordnete Membran (30) aufweist und über dem im Filtergehäuse (12) angeordneten Filterkörper (14) ein Freiraum (48) gebildet ist, der der Membran (30) eine auf den Filterkörper (14) zugewandte Bewegung beim Einatmen ermöglicht.

2. Feuchte- und Wärmeaustauscher gemäß Anspruch 1, **dadurch gekennzeichnet, dass** Haltemittel (22) auf dem Boden (26) des Filtergehäuses (12) angeordnet sind.

3. Feuchte- und Wärmeaustauscher gemäß einem der vorhergehenden Ansprüche, **dadurch gekennzeichnet, dass** das Haltemittel (22) als auf der Innenumfangsfläche (24) angeordneter umlaufender Ringwulst ausgebildet ist.

4. Feuchte- und Wärmeaustauscher gemäß einem der vorhergehenden Ansprüche, **dadurch gekennzeichnet, dass** das Haltemittel (22) zapfenförmig ausgebildet ist.

5. Feuchte- und Wärmeaustauscher gemäß Anspruch 4, **dadurch gekennzeichnet, dass** mindestens zwei zapfenförmige Haltemittel (22) im Filtergehäuse (12) angeordnet sind.

6. Feuchte- und Wärmeaustauscher gemäß einem der vorhergehenden Ansprüche, **dadurch gekennzeichnet, dass** das Haltemittel (22) auf den Filterkörper (14) hin sich verjüngend ausgebildet ist.

7. Feuchte- und Wärmeaustauscher gemäß einem der vorhergehenden Ansprüche, **dadurch gekennzeichnet, dass** das Haltemittel (22) hakenförmig ausgebildet ist.

8. Feuchte- und Wärmeaustauscher gemäß einem der vorhergehenden Ansprüche, **dadurch gekennzeichnet, dass** der Deckelteil (28) eine von der Membran (30) überdeckte Öffnung (32) aufweist.

9. Feuchte- und Wärmeaustauscher gemäß einem der vorhergehenden Ansprüche, **dadurch gekennzeichnet, dass** die Membran (30) auf der Innenseite (40) des Deckelteiles (28) an mindestens einem Befestigungspunkt (34) gehalten ist.

10. Feuchte- und Wärmeaustauscher gemäß einem der vorhergehenden Ansprüche, **dadurch gekennzeichnet, dass** die Membran (30) eine unebene Oberfläche aufweist.

## Claims

1. A moisture and heat exchanger (10) with a speaking function, in particular for single use, having a filter housing (12) with a base (26) and with a filter body (14) at least partially arranged therein, wherein the filter housing has a speaking valve (16) arranged on its distal side (18), **characterised in that** at least one holding means (22) to hold the filter body (14) is arranged in the interior (13) of the filter housing (12), the holding means (22) being arranged on an inner circumferential surface (24) of the filter housing (12) and engaging the filter body (14), the speaking valve (16) having a membrane (30) arranged in a cover part (28) and a free space (48) being formed via the filter body (14) arranged in the filter housing (12), which free space (48) makes it possible for the membrane (30) to make a movement towards the filter body (14) when inhalation takes place.

2. A moisture and heat exchanger according to claim 1, **characterised in that** holding means (22) is arranged on the base (26) of the filter housing (12).

3. A moisture and heat exchanger according to any one of the preceding claims, **characterised in that** the holding means (22) is in the form of a circumferential annular bulge arranged on the inner circumferential surface (24).

4. A moisture and heat exchanger according to any one of the preceding claims, **characterised in that** the holding means (22) is peg-shaped.

5. A moisture and heat exchanger according to claim 4, **characterised in that** at least two peg-shaped holding means (22) are arranged in the filter housing (12).

6. A moisture and heat exchanger according to any one of the preceding claims, **characterised in that** the holding means (22) tapers towards the filter body (14).

7. A moisture and heat exchanger according to any one of the preceding claims, **characterised in that** the holding means (22) is hook-shaped.

8. A moisture and heat exchanger according to any one of the preceding claims, **characterised in that** the cover part (28) has an opening (32) covered by the membrane (30).

9. A moisture and heat exchanger according to any one of the preceding claims, **characterised in that** the membrane (30) is held on the inner surface (40) of the cover part (28) at at least one attachment point (34).

10. A moisture and heat exchanger according to any one of the preceding claims, **characterised in that** the membrane (30) has an uneven surface.

## Revendications

1. Échangeur d'humidité et de chaleur (10) à fonction vocale, en particulier pour un usage unique, comportant un boîtier de filtrage (12) avec un fond (26) et un corps de filtrage (14), disposé au moins partiellement dans celui-ci, le boîtier de filtrage comportant une valve vocale (16), agencée sur le côté distal (18) de celui-ci, **caractérisé en ce qu'**à l'intérieur (13) du boîtier de filtrage (12) est disposé au moins un moyen de fixation (22) destiné à maintenir le corps de filtrage (14), le moyen de fixation (22) étant disposé sur une surface périphérique intérieure (24) du boîtier de filtrage (12) et s'engageant à l'intérieur du corps de filtrage (14), la valve vocale (16) comportant une membrane (30) disposée dans une partie formant couvercle (28) et un dégagement (48) étant formé au-dessus du corps de filtrage (14) disposé dans le boîtier de filtrage (12), lequel permet à la membrane (30) d'effectuer un mouvement vers le corps de filtrage (14) pendant une inspiration.

2. Échangeur d'humidité et de chaleur selon la revendication 1, **caractérisé en ce que** des moyens de fixation (22) sont disposés sur le fond (26) du boîtier de filtrage (12).

3. Échangeur d'humidité et de chaleur selon l'une quelconque des revendications précédentes, **caractérisé en ce que** le moyen de fixation (22) est réalisé sous la forme d'un bourrelet annulaire disposé sur le pourtour de la surface périphérique intérieure (24).

4. Échangeur d'humidité et de chaleur selon l'une quelconque des revendications précédentes, **caractérisé en ce que** le moyen de fixation (22) est réalisé sous forme de tenon.

5. Échangeur d'humidité et de chaleur selon la revendication 4, **caractérisé en ce qu'**au moins deux moyens de fixation (22) en forme de tenon sont disposés dans le boîtier de filtrage (12).

6. Échangeur d'humidité et de chaleur selon l'une quelconque des revendications précédentes, **caractérisé en ce que** le moyen de fixation (22) est réalisé en se rétrécissant vers le corps de filtrage (14).

7. Échangeur d'humidité et de chaleur selon l'une quelconque des revendications précédentes, **caractérisé en ce que** le moyen de fixation (22) est réalisé sous forme de crochet.

8. Échangeur d'humidité et de chaleur selon l'une quelconque des revendications précédentes, **caractérisé en ce que** la partie formant couvercle (28) comporte une ouverture (32) recouverte par la membrane (30).

9. Échangeur d'humidité et de chaleur selon l'une quelconque des revendications précédentes, **caractérisé en ce que** la membrane (30) est maintenue sur la face intérieure (40) du couvercle (28) en au moins un point de fixation (34).

10. Échangeur d'humidité et de chaleur selon l'une quelconque des revendications précédentes, **caractérisé en ce que** la membrane (30) comporte une surface non plane.
